# EUROPÄISCHE PATENTSCHRIFT

(11) **EP 0 410 958 B1**
(45) Veröffentlichungstag und Bekanntmachung des Hinweises auf die Patenterteilung: **27.01.1993**
(21) Anmeldenummer: 90890223.2
(22) Anmeldetag: 25.07.1990
(51) Int. Cl.: A61K 31/42, A61K 9/00, A61K 47/22

(54) **Verfahren zur Herstellung stabiler Injektionspräparate**
Process to prepare stable injectable compositions
Procédé de préparation de compositions injectables stables

(30) Priorität: 26.07.1989 AT 1804/89
(43) Veröffentlichungstag der Anmeldung: 30.01.1991
(73) Patentinhaber: F. Joh. Kwizda Unternehmens- Verwaltungsgesellschaft m.b.H., A-1011 Wien (AT); TAIHO PHARMACEUTICAL CO., LTD., Tokyo 101 (JP)
(72) Erfinder: Farkouh, Edmond, Dr. phil.et Mag. pharm., A-1220 Wien (AT)
(74) Vertreter: Müllner, Erwin, Dr.

(56) Entgegenhaltungen:
- EP-A- 0 026 928
- EP-A- 0 052 962
- STN INTERNATIONAL INFORMATION SERVICES DATENBANK: CHEMICAL ABSTRACTS, Zugangsnummer 103, Nr. 13, Zusammenfassung Nr. 104953z
- H.P.Fiedler: Lexicon der Hilfsstoffe für Pharmazie, Kosmetik und angrenzende Gebiete, 3 Auflage, S. 577(Band 1)

## Beschreibung

Die Erfindung bezieht sich auf ein Verfahren zur Herstellung stabiler wässeriger und/oder propylenglykolischer Injektionspräparate, die neben pharmazeutisch unbedenklichen für parenterale Verabreichung geeigneten Stoffen als Wirkstoff eine Verbindung der allgemeinen Formel 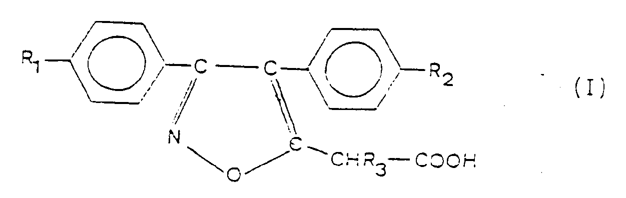
worin R₁ und R₂ unabhängig voneinander je eine verzweigte oder unverzweigte C₁-C₄-Alkoxygruppe und R₃ Wasserstoff oder eine C₁-C₄-Alkylgruppe bedeuten, oder ein pharmazeutisch unbedenkliches Salz davon, insbesondere 3,4-Di-(p-methoxyphenyl)-isoxazol-5-essigsäure, enthalten.

Verbindungen der allgemeinen Formel sind in der EP-B-26 928 beschrieben. Die Verbindungen der Formel I und deren Salze besitzen analgetische, antipyretische und antiinflammatorische Wirkungen.

Die Herstellung von stabilen wässerigen und/oder propylenglykolischen Injektionslösungen der geannten Verbindungen ist insofern auf Schwierigkeiten gestoßen, als einerseits die Verbindungen der Formel I in Wasser bzw. Proypenglykol schwer löslich sind und anderseits aus den Lösungen beim Lagern der Wirkstoff auskristallisiert.

Ziel der Erfindung ist daher die Herstellung von stabilen wässerigen und/oder propylenglykolischen, eine Verbindung der Formel I oder ein pharmazeutischen annehmbares Salz davon enthaltenden Injektionspräparaten.

Dieses Ziel wird mit einem Verfahren der eingangs genannten Art erreicht, bei dem erfindungsgemäß der Wirkstoff in Tetrahydrofurfurylalkoholpolyethylenglykolether oder in einer Mischung aus Propylenglykol und Tetrahydrofurfurylalkoholpolyethylenglykolether oder Wasser und Tetrahydrofurfurylalkoholpolyethylenglykolether gelöst bzw. suspendiert, bei Vorliegen einer Suspension diese durch Zugabe einer Base in eine Lösung übergeführt und gegebenenfalls die erhaltene Lösung mit Wasser verdünnt wird, wobei in der aus Tetrahydrofurfurylalkoholpolyethylenglykolether und Wasser und/oder Propylenglykol bestehenden Mischung der Tetrahydrofurfurylalkoholpolyethylenglykolether in einer Menge von 10 bis 50, vorzugsweise 15 bis 30, Vol-% vorliegt, und gewünschtenfalls der pH-Wert der Injektionspräparate durch Zugabe von pharmazeutisch unbedenklichen Säuren oder Basen auf 3,0 bis 12,0 eingestellt wird.
Es wurde überraschenderweise gefunden, daß durch die Gegenwart von Tetrahydrofurfurylalkoholpolyethylenglykolether stabile Injektionspräparate erhalten werden, bei denen auch bei längerer Lagerung keine Auskristallisation erfolgt.

In den erfindungsgemäß hergestellten Injektionspräpraten beträgt die bevorzugte Konzentration an einer Verbindung der Formel (I) bzw. deren Salz berechnet auf die Stubstanz der Formel (I) 25 - 100 mg/ml fertige Lösung. Als Verbindung der Formel (I) wird vorzugsweise 3,4-Di-(p-methoxyphenyl)-isoxazol-5-essigsäure eingesetzt. Verwendbare Salze von Verbindungen der Formel (I) sind alle pharmazeutisch annehmbaren Salze, die keine höhere Toxizität als die freie Säure besitzen. Bevorzugte Salze von Verbindungen der Formel (I) sind das Natriumsalz und des Kaliumsalz.

Als Lösungsmittel werden Wasser und/oder Propylenglykol in Mischung mit Tetrahydrofurfurylalkoholpolyethylenglykolether verwendet, wobei der Anteil an Tetrahydrofurfurylalkoholpolyethylenglykolether 50, insbesonder 30, Vol-% nicht übersteigt.

Der pH-Wert der erfindungsgemäß hergestellten Injektionspräparate wird in der Regel mit Natronlauge auf 3,0 bis 12,0, insbesondere 4 bis 8, eingestellt. Die erfindungsgemäß hergestellten Injektionspräparate können im Bedarfsfall mit NaCl oder einer anderen pharmakologisch unbedenklichen Hilfssubstanz isotonisiert werden.

In der Praxis erfolgt die erfindungsgemäße Herstellung einer Injektionslösung beispielsweise dadurch, daß eine Verbindung der Formel (I) in der Gesamtmenge Tetrahydrofurfurylalkoholpolyethylenglykolether und gegebenenfalls Propylenglykol suspendiert und durch Zugabe von Natronlauge (c = 10 Mol/l) gelöst wird, wobei die Menge an Natronlauge maximal 90 % der für die Salzbildung stöchiometrisch notwendigen Menge entspricht. Dann wird mit der gewünschten Menge Wasser aufgefüllt und gegebenenfalls der pH mit Natronlauge (c = 1 Mol/g) eingestellt. Dann wird die Lösung mit Wasser auf das gewünschten endgültige Volumen aufgefüllt, filtriert und anschließend bei 121°C im Autoklaven 30 min lang sterilisiert.

Zur Herstellung einer wasserfreien Injektionslösung wird die erforderliche Menge an z. B. Natronlauge in fester Form zugegeben. Die Injektionslösung kann durch Zugabe von beispielsweise Kochsalz isotonisiert werden.

Die Wirkstoffe der allgemeinen Formel (I) können nach dem in der EP-PS 26 928 beschriebenen Verfahren erhalten werden.

### BEISPIEL 1:

3,4-Di-(p-methoxyphenyl)-isoxazol-5-essigsäure wurde in Tetrahydrofurfurylalkoholpolyethylenglykolether suspendiert und durch Zugabe von NaOH gelöst. Anschließend wurde Wasser zugegeben und der pH-Wert auf 6,5 eingestellt. Das Volumenverhältnis von Wasser zu Tetrahydrofurfurylalkoholpolyethylenglykolether betrug 70:30; Endkonzentration an Wirkstoff 80 mg/ml.

### BEISPIEL 2:

3,4-Di-(p-methoxyphenyl)-isoxazol-5-essigsäure wurde in einer Mischung aus Tetrahydrofurfurylalkoholpolyethylenglykolether und Propylenglykol suspendiert und durch Zugabe von NaOH gelöst. Anschließend wurde Wasser zugegeben und der pH Wert auf 7,0 eingestellt. Das Volumenverhältnis von Wasser zu Tetrahydrofurfurylalkoholpolyethylenglykolether zu Propylenglykol betrug 50:20:30; Endkonzentration an Wirkstoff 50 mg/ml.

### BEISPIEL 3:

3,4-Di-(p-methoxyphenyl)-isoxal-5-essigsäure wurde in einer Mischung aus Tetrahydrofurfurylalkoholpolyethylenglykolether und Propylenglykol suspendiert und durch Zugabe von NaOH gelöst. Anschließend wurde Wasser zugegeben und der pH Wert auf 12 eingestellt. Das Volumenverhältnis von Wasser zu Tetrahydrofurfurylalkoholpolyethylenglykolether zu Propylenglykol betrug 50:30:20; Endkonzentration an Wirkstoff 100 mg/ml.

### BEISPIEL 4:

3,4-Di(p-methoxyphenyl)-isoxazol-5-essigsäure wurde in einer Mischung aus Tetrahydrofurfurylalkoholpolyethylenglykolether und Propylenglykol suspendiert und durch Zugabe von NaOH gelöst. Anschließend wurde Wasser zugegeben und der pH-Wert auf 5,5 eingestellt. Das Volumenverhältnis von Wasser zu Tetrahydrofurfurylalkoholpolyethylenglykolether zu Propylenglykol betrug 50:30:20; Endkonzentration an Wirkstoff 70 mg/ml.

### BEISPIEL 5:

3,4-Di-(p-methoxyphenyl)-isoxazol-5-essigsäure wurde in einer Mischung aus Tetrahydrofurfurylalkoholpolyethylenglykolether und Propylenglykol suspendiert und durch Zugabe von NaOH gelöst. Anschließend wurde Wasser zugegeben und der pH-Wert auf 7 bis 8 eingestellt. Das Volumenverhältnis von Wasser zu Tetrahydrofurfurylalkoholpolyethylenglykolether zu Propylenglykol betrug 50:30:20; Endkonzentration an Wirkstoff 85 mg/ml.

### BEISPIEL 6:

3,4-Di-(p-methoxyphenyl)-isoxazol-5-essigsäure wurde in einer Mischung aus Tetrahydrofurfurylalkoholpolyethylenglykolether und Propylenglykol suspendiert und durch Zugabe von NaOH gelöst. Anschließend wurde Wasser zugegeben und der pH-Wert auf 4 bis 5,5 eingestellt. Das Volumenverhältnis von Wasser zu Tetrahydrofurfurylalkoholpolyethylenglykolether zu Propylenglykol betrug 60:25:15; Endkonzentration an Wirkstoff 60 mg/ml.

Es wurde überraschenderweise gefunden, daß durch die Gegenwart von Tetrahydrofurfurylalkoholpolyethylenglykolether stabile Injektionspräparate erhalten werden, bei denen auch bei längerer Lagerung keine Auskristallisation erfolgt.

## Patentansprüche

1. Verfahren zur Herstellung stabiler wässeriger und/oder propylenglykolischer Injektionspräparate, die neben pharmazeutisch unbedenklichen für parenterale Verabreichung geeigneten Stoffen als Wirkstoff eine Verbindung der allgemeinen Formel 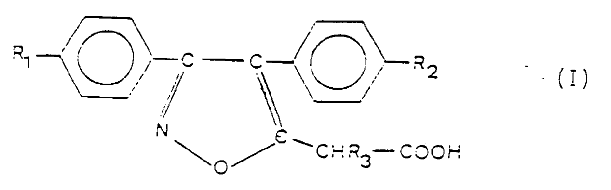 worin R₁ und R₂ unabhängig voneinander je eine verzweigte oder unverzweigte C₁-C₄-Alkoxygruppe und R₃ Wasserstoff oder eine C₁-C₄-Alkylgruppe bedeuten, oder ein pharmazeutisch unbedenkliches Salz davon, insbesondere 3,4-Di-(p-methoxyphenyl)-isoxazol-5-essigsäure, enthalten, dadurch gekennzeichnet, daß der Wirkstoff in Tetrahydrofurfurylalkoholpolyethylenglykolether oder in einer Mischung aus Propylenglykol und Tetrahydrofurfurylalkoholpolyethylenglykolether oder Wasser und Tetrahydrofurfurylalkoholpolyethylenglykolether gelöst bzw. suspendiert, bei Vorliegen einer Suspension diese durch Zugabe einer Base in eine Lösung übergeführt und gegebenenfalls die erhaltene Lösung mit Wasser verdünnt wird, wobei in der aus Tetrahydrofurfurylalkoholpolyethylenglykolether und Wasser und/oder Propylenglykol bestehenden Mischung der Tetrahydrofurfurylalkoholpolyethylenglykolether in einer Menge von 10 bis 50, vorzugsweise 15 bis 30, Vol-% vorliegt, und gewünschtenfalls der pH-Wert der Injektionspräparate durch Zugabe von pharmazeutisch unbedenklichen Säuren oder Basen auf 3,0 bis 12,0 eingestellt wird.

2. Verfahren nach Anspruch 1, dadurch gekennzeichnet, daß zur Überführung der Suspension in eine Lösung Natron- oder Kalilauge eingesetzt wird.

3. Verfahren nach Anspruch 1 oder 2, dadurch gekennzeichnet, daß der pH-Wert der Präparate auf 4 bis 8 eingestellt wird.

## Claims

1. A method for the production of stable aqueous and/or propylene glycol injection preparations, which in addition to substances which are pharmaceutically harmless and suited to being administered parenterally, contain as active ingredient a compound of the general formula 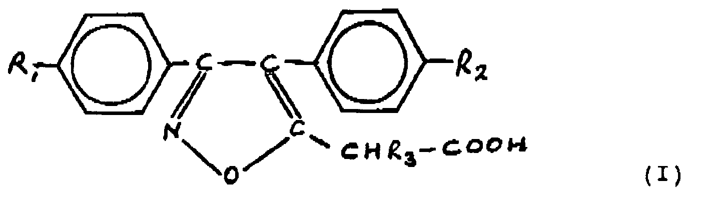 in which R₁ and R₂ independently of each other each denote a branched or unbranched C₁-C₄ alkoxy group and R₃ denotes hydrogen or a C₁-C₄ alkyl group, or a pharmaceutically harmless salt thereof, in particular 3,4-di-(p-methoxyphenyl)-isoxazol-5-acetic acid, characterised in that the active ingredient is dissolved or suspended in tetrahydrofurfuryl alcohol polyethylene glycol ether or in a mixture of propylene glycol and tetrahydrofurfuryl alcohol polyethylene glycol ether or water and tetrahydrofurfuryl alcohol polyethylene glycol ether, in the presence of a suspension this is converted into a solution through the addition of a base and if necessary the obtained solution is diluted with water, in which in the mixture consisting of tetrahydrofurfuryl alcohol polyethylene glycol ether and water and/or propylene glycol, the tetrahydrofurfuryl alcohol polyethylene glycol ether is present in an amount of 10 to 50, preferably 15 to 30 vol. %, and if desired the pH value of the injection preparations is set at 3.0 to 12.0 through the addition of pharmaceutically harmless acids or bases.

2. A method according to Claim 1, characterised in that for the conversion of the suspension into a solution, caustic soda or caustic potash is used.

3. A method according to Claim 1 or 2, characterised in that the pH value of the preparations is adjusted to 4 to 8.

## Revendications

1. Procédé de préparation de compositions injectables stables dans l'eau et/ou le propylèneglycol qui, en tant que substances actives, outre des substances appropriées à l'administration par voie parentérale et pharmaceutiquement compatibles, contiennent un dérivé de formule générale: 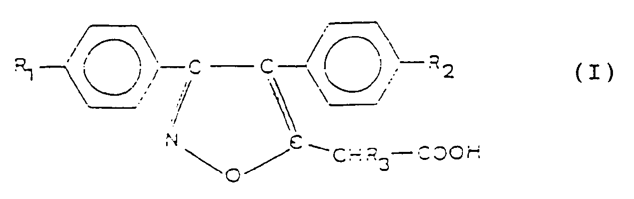 dans laquelle:
R₁ et R₂ indépendamment l'un de l'autre, signifient chaque fois un groupe alkoxy en C₁ à C₄ ramifié ou non ramifié; et
R₃ est un atome d'hydrogène ou un groupe alkyle en C₁ à C₄,
ou un de leurs sels pharmaceutiquement compatibles, notamment l'acide 3,4-di-(p-méthoxyphényl)-isoxazol-5-acétique,
caractérisé en ce que la substance est dissoute et/ou mise en suspension dans du polyéthylèneglycoléther de l'alcool tétrahydrofurfuryle dans un mélange de propylèneglycol et de polyéthylèneglycoléther de l'alcool tétrahydrofurfuryle ou d'eau et de polyéthylèneglycoléther de l'alcool tétrahydrofurfuryle, en présence d'une suspension, on met celle-ci en solution par addition d'une base et, le cas échéant, dilue la solution obtenue dans de l'eau, le polyéthylèneglycoléther de l'alcool tétrahydrofurfuryle étant, dans le mélange constitué de polyéthylèneglycoléther de l'alcool tétrahydrofurfuryle et d'eau et/ou de propylèneglycol présent en quantité de 10 à 50, de préférence de 15 à 30% en volume, et le pH de la composition injectable étant, si on le souhaite, porté à une valeur de 3,0 à 12,0 par addition d'acide ou de base pharmaceutiquement compatible.

2. Procédé selon la revendication 1, caractérisé en ce que, pour transformer la suspension en solution, on utilise de la potasse ou de la soude caustique.

3. Procédé selon la revendication 1 ou 2, caractérisé en ce que le pH de la préparation est réglé à une valeur comprise entre 4 et 8.
